# EUROPEAN PATENT APPLICATION

(11) **EP 4 435 086 A1**
(43) Date of publication of application: **25.09.2024**
(21) Application number: 22895669.4
(22) Date of filing: 17.11.2022
(51) Int. Cl.: C12M 3/00, C12N 5/071, C12N 1/20, C12Q 1/02, C12M 1/00, C12M 1/34

(54) **CELL CULTURE APPARATUS, KIT FOR CELL CULTURE, AND CELL CULTURE METHOD**

(30) Priority: 18.11.2021 JP 2021187641
(71) Applicant: FUJIFILM Corporation, Tokyo 106-8620 (JP)
(72) Inventor: YAMAZAKI, Nao, Ashigarakami-gun, Kanagawa 258-8577 (JP); SHIGESADA, Keiji, Ashigarakami-gun, Kanagawa 258-8577 (JP); WAKABAYASHI, Akira, Ashigarakami-gun, Kanagawa 258-8577 (JP); MIMA, Shinji, Ashigarakami-gun, Kanagawa 258-8577 (JP); IMAKURA, Yuki, Ashigarakami-gun, Kanagawa 258-8577 (JP)
(74) Representative: Hoffmann Eitle
(86) International application number: PCT/JP2022/042675
(87) International publication number: WO 2023/090388

(57) **Abstract**

An object of the present invention is to provide a cell culture device capable of reproducing, in vitro, a growth and proliferation environment of an anaerobic bacterium in a living body; a kit for cell culture; and a cell culture method. According to the present invention, there is provided a cell culture device including a plate having a concave portion for accommodating a culture medium, a cell accommodating container having a gas permeable membrane, a first member for holding the cell accommodating container, and a second member having a structure for regulating a gas concentration in an upper space of the cell accommodating container, in which, in a case where a cell sheet is formed on an upper surface of the gas permeable membrane, a space above the gas permeable membrane is in a sealed state in which gas does not pass through.

## Description

### Technical Field

The present invention relates to a cell culture device, a kit for cell culture, and a cell culture method.

### Background Art

Intestinal bacteria are a general term for bacteria which are constantly present in the intestine of animals including humans. Since the inside of the intestine is under anaerobic conditions, it is considered that many of the intestinal bacteria are anaerobic bacteria. Intestinal bacteria play a role in the digestion of ingested food by absorbing various substances present in the intestine and excreting useful metabolites into the intestine. In addition, the intestinal bacteria play a role of preventing the growth of an exogenous pathogenic bacterium invading the intestine and preventing the onset of a disease caused by the pathogenic bacterium by being present in the intestine. On the other hand, the intestinal bacteria may produce harmful substances as metabolites, which have a harmful effect on animals, and these harmful substances may directly act on the intestine of the animals or may be absorbed from the intestine, thereby causing disorders or diseases.

As described above, it has been known that the intestinal bacteria have a significant influence on the health and diseases of the animals including humans, and there is an increasing demand for an in vitro evaluation system using the intestinal bacteria in the development of functional foods and pharmaceuticals.

Many of the intestinal bacteria can be maintained and proliferated only in an anaerobic environment. In a case where the intestinal bacteria are cultured alone, the intestinal bacteria can be cultured by placing a culture container in the anaerobic environment. However, in the in vitro evaluation system using the intestinal bacteria, there is a demand for co-culture of intestinal epithelial cells and intestinal bacteria in order to evaluate influence of the intestinal bacteria on the living body. In order to perform the co-culture of the intestinal epithelial cells and the intestinal bacteria, it is necessary to maintain the intestinal epithelial cells in an aerobic environment and to provide an anaerobic environment to the intestinal bacteria.

As a method for providing aerobic conditions to the intestinal epithelial cells and providing anaerobic conditions to the intestinal bacteria, Patent Document 1 discloses a culture system for co-culturing a first cell group with a cell layer or tissue formed of a second cell group different from the first cell group, the culture system including a first culture tank in which the first cell group is co-cultured with the cell layer or tissue formed of the second cell group under anaerobic conditions, a second culture tank in which a culture solution is stored under aerobic conditions, and a substance exchange structure which is disposed to connect the first culture tank and the second culture tank to each other and holds the cell layer or tissue to cover a surface of the first culture tank side.

### Prior Art Documents

### Patent Documents

Patent Document 1: WO2018/079793A

### SUMMARY OF THE INVENTION

### Object to be solved by the invention

The device disclosed in Patent Document 1 has a configuration that a space of a lower groove of a cell culture insert on which membranous intestinal epithelial cells are cultured is sealed as an airtight structure, this space is filled with a culture medium containing abundant dissolved oxygen, and the entire container is put into an anaerobic chamber or the like to maintain an upper side with a free space in an anaerobic state. However, the device disclosed in Patent Document 1 has a problem that a special and expensive culture tank, such as the anaerobic chamber, is required, and a problem that the cost of the culture medium to be used is increased in order to prevent a decrease in dissolved oxygen concentration of the culture medium put into the sealed space.

An object of the present invention is to provide a cell culture device capable of reproducing, in vitro, a growth and proliferation environment of an anaerobic bacterium (for example, anaerobic intestinal bacteria) in a living body without requiring a special and expensive culture tank such as an anaerobic chamber. Another object of the present invention is to provide a kit for cell culture, including the cell culture device, and a cell culture method using the cell culture device.

### Means for solving the object

As a result of intensive studies to solve the above-described problems, the present inventor has found that the above-described problems can be solved by, in a cell culture device including a plate having a concave portion for accommodating a culture medium, a cell accommodating container having a gas permeable membrane, a first member for holding the cell accommodating container, and a second member having a structure for regulating a gas concentration in an upper space of the cell accommodating container, in a case where a cell sheet is formed on an upper surface of the gas permeable membrane, making a space above the gas permeable membrane to be in a sealed state in which gas does not pass through. The present invention has been completed based on the above findings.
(1) A cell culture device comprising:
   a plate having a concave portion for accommodating a culture medium;
   a cell accommodating container having a gas permeable membrane;
   a first member for holding the cell accommodating container; and
   a second member having a structure for regulating a gas concentration in an upper space of the cell accommodating container,
   in which, in a case where a cell sheet is formed on an upper surface of the gas permeable membrane, a space above the gas permeable membrane is in a sealed state in which gas does not pass through.
(2) The cell culture device according to (1),
   in which the cell accommodating container is attachable and detachable.
(3) The cell culture device according to (1) or (2),
   in which the space above the gas permeable membrane is allowed to be accessed by removing the second member.
(4) The cell culture device according to any one of (1) to (3),
   in which the structure for regulating the gas concentration in the upper space of the cell accommodating container is a structure for holding an oxygen scavenger and/or a structure for supplying gas having an adjusted concentration.
(5) The cell culture device according to any one of (1) to (4),
   in which the second member has a structure for measuring the gas concentration in the upper space.
(6) A kit for cell culture, comprising:
   the cell culture device according to any one of (1) to (5); and
   an oxygen scavenger.
(7) A cell culture method comprising:
   culturing cells on the gas permeable membrane in a state in which the cells form a cell sheet, using the cell culture device according to any one of (1) to (5).
(8) The cell culture method according to (7),
   in which the cells are intestinal epithelial cells.
(9) The cell culture method according to (7) or (8),
   in which bacteria are co-cultured on the cell sheet.
(10) The cell culture method according to (9),
   in which the bacteria are bacteria capable of inhabiting a human intestine.
(11) The cell culture method according to (9) or (10),
   in which the cells are cultured in aerobic conditions, and the bacteria are cultured in anaerobic conditions.
(12) The cell culture method according to any one of (9) to (11),
   in which a response of intestinal epithelial cells or bacteria is evaluated by co-culturing the intestinal epithelial cells and the bacteria.
(13) The cell culture method according to any one of (7) to (12),
   in which the cells are cultured by installing the cell culture device according to any one of (1) to (5) in a CO₂ gas incubator or in a multi-gas incubator.

### Effect of the invention

According to the present invention, it is possible to perform co-culture of aerobic cells and anaerobic bacteria without using an anaerobic chamber.

### BRIEF DESCRIPTION OF THE DRAWINGS

Fig. 1 is a view of an external apparatus and cross section of a cell culture device.
Fig. 2 is an exploded view showing an overall assembly of the cell culture device.
Fig. 3 is an enlarged view showing a main part of the cell culture device in an enlarged manner.
Fig. 4 is views showing a state during an exchange of a culture medium, a state during a culture, and a state during a cell observation and a chemical liquid injection.
Fig. 5 is a view showing a state during insertion and removal of an insert.
Fig. 6 is a graph showing an oxygen concentration in a case where an oxygen scavenger is not used.
Fig. 7 is a graph showing an oxygen concentration after Anaeropouch is installed in an upper tank of the device.
Fig. 8 is a graph showing an oxygen concentration after Anaeropack is installed in an upper tank of the device.
Fig. 9 is a graph showing an oxygen concentration in the upper tank after a cell culture insert seeded with cells is installed in the cell culture device and the upper tank including the Anaeropack is sealed.
Fig. 10 is a graph showing an oxygen concentration in a lower tank after a cell culture insert seeded with cells is installed in the cell culture device and the upper tank including the Anaeropack is sealed.
Fig. 11 is a graph showing a trans-epithelial electrical resistance (TEER) value of intestinal epithelial cells after 72 hours of culture in the cell culture device.
Fig. 12 shows the number of bacteria (left figure) and the survival rate (right figure) after 48 hours of co-culture of the intestinal epithelial cells and the bacteria (B. fragilis: upper row, L. lactis: lower row).

### DESCRIPTION OF THE PREFERRED EMBODIMENTS

Embodiments of the present invention will be described below.

In the present specification, a term "anaerobic conditions" or "anaerobic state" refers to conditions or a state, in which oxygen in a medium is present at a low concentration or is absent. An oxygen concentration set in the "anaerobic conditions" or "anaerobic state" can be appropriately set by those skilled in the art, depending on the type of bacteria to be used. For example, the "anaerobic conditions" or "anaerobic state" is a state in which the oxygen concentration in the gas is 0% or more and less than 5%, but is not particularly limited.

In the present specification, a term "aerobic conditions" or "aerobic state" refers to conditions or a state, in which oxygen in a medium is present at a high concentration or the entire medium is oxygen. An oxygen concentration set in the "aerobic conditions" or "aerobic state" can be appropriately set by those skilled in the art, depending on the type of bacteria to be used. For example, the "aerobic conditions" or "aerobic state" is a state in which the oxygen concentration in the gas is 10% to 25%, or a dissolved oxygen concentration in a culture solution can be achieved at 8.26 mg/L under conditions of 1 atm and 25°C, but is not limited thereto.

### <Cell culture device>

The cell culture device according to the embodiment of the present invention is a device including a plate having a concave portion for accommodating a culture medium, a cell accommodating container having a gas permeable membrane, a first member (first chamber) for holding the cell accommodating container, and a second member (second chamber) having a structure for regulating a gas concentration in an upper space of the cell accommodating container, in which, in a case where a cell sheet is formed on an upper surface of the gas permeable membrane, a space above the gas permeable membrane is in a sealed state in which gas does not pass through. As described above, in a case where the cell sheet is formed on the upper surface of the gas permeable membrane, the space above the gas permeable membrane is in a sealed state in which the gas does not pass through, so that the cell sheet can be cultured in the aerobic state, and bacteria seeded on the cell sheet can be cultured in the anaerobic state.

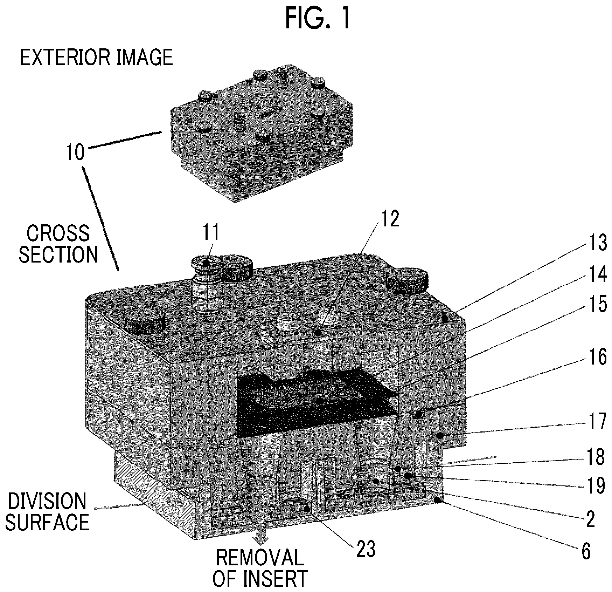

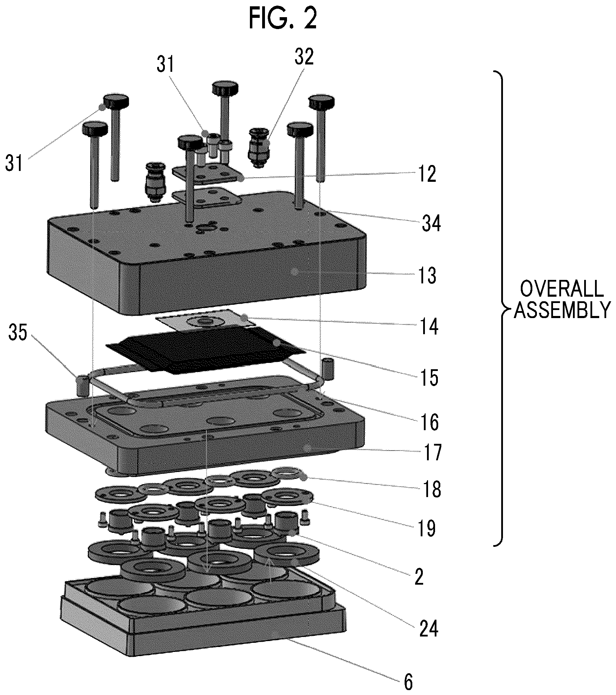

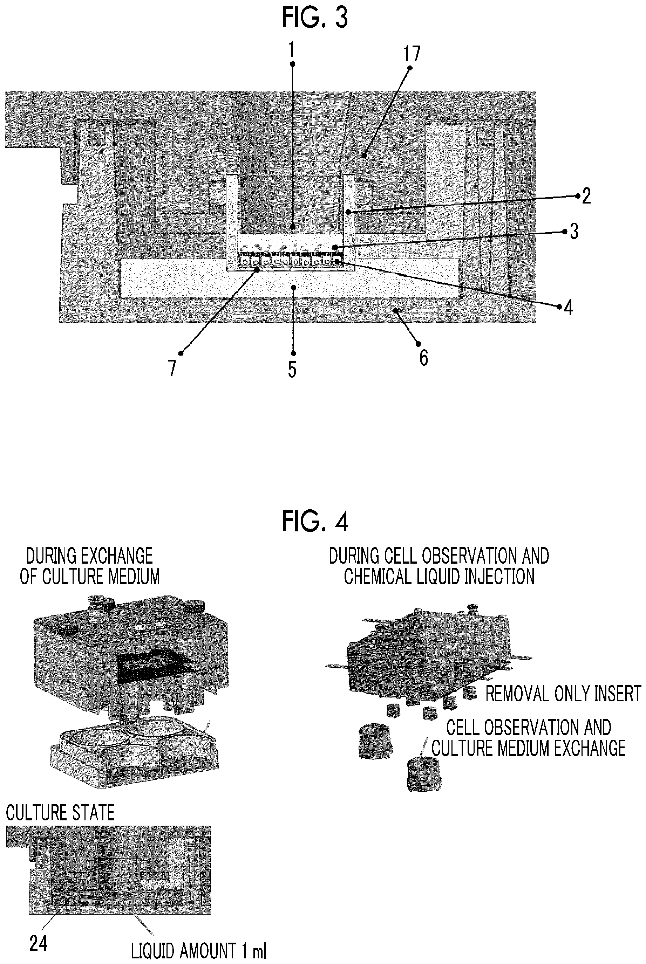

Figs. 1 to 3 are diagrams showing an example of the cell culture device according to the embodiment of the present invention. Fig. 1 is a view of an external apparatus and cross section of the cell culture device, Fig. 2 is an exploded view showing an overall assembly of the cell culture device, and Fig. 3 is an enlarged view showing a main part of the cell culture device in an enlarged manner.

A cell culture device 10 according to the present embodiment includes a plate 6 having a concave portion for accommodating a culture medium. The plate 6 is disposed below a cell accommodating container (insert) 2 described later, and forms an outer shape of the device lower part.

The plate preferably has a plurality of concave portions for accommodating the culture medium. The plurality of portions are not particularly limited as long as there are two or more portions, and the number thereof is preferably 2 or more and 100 or less, more preferably 2 or more and 50 or less, and particularly preferably 2 or more and 40 or less.

A form of the concave portion included in the plate is not particularly limited as long as the concave portion can accommodate the culture medium. The cell accommodating container is also accommodated in the concave portion. Therefore, it is preferable that, in the concave portion of the plate, an opening portion of the plate is larger than a diameter of an opening portion of the cell accommodating container. Specifically, the opening portion of the plate is larger than a circle-equivalent diameter of the opening portion of the cell accommodating container by preferably 2 mm or more and more preferably 5 mm or more. The upper limit thereof is not particularly limited, but is practically 20 mm or less from the circle-equivalent diameter of the opening portion of the cell accommodating container. It is preferable that a depth of the concave portion of the plate is a dimension in which the above-described cell accommodating container can be accommodated. The depth of the concave portion is larger than a depth of the cell accommodating container by preferably 2 mm or more and more preferably 5 mm or more. The upper limit thereof is not particularly limited, but is practically 20 mm or less therefrom.

The cell culture device 10 according to the present embodiment includes the cell accommodating container 2 having a gas permeable membrane (porous membrane) 7. It is preferable that the cell culture device 10 includes a plurality of the cell accommodating containers 2. Here, the number of containers is not particularly limited as long as there are two or more containers, but is preferably 2 or more and 100 or less, more preferably 2 or more and 50 or less, and particularly preferably 2 or more and 40 or less.

The cell accommodating container is not particularly limited as long as it is a container capable of accommodating cells, but a container having a function of culturing the cells in the container is preferable. As the cell accommodating container, for example, a cell culture container also referred to as a cell culture insert (or insert) may be used. A shape of the cell accommodating container is not particularly limited, and examples thereof include a container having a substantially cylindrical shape. In the cell accommodating container having a substantially cylindrical shape, a circle-equivalent diameter of an opening portion is preferably 3 mm or more, and more preferably 10 mm or more. The upper limit value thereof is practically 35 mm or less.

The insert 2 is disposed on an upper part of the plate 6 and has a cylindrical shape having no upper end. In the illustrated example, six inserts 2 can be installed in the plate 6 (6-well plate). A bottom surface of the insert 2 is a gas permeable membrane (for example, a porous membrane), and the cell culture insert 2 has a configuration in which only gas in the upper tank and gas in the lower tank can be exchanged. In a case where a cell sheet is formed on the upper surface of the gas permeable membrane, the cell sheet serves as a barrier, and thus a configuration in which the upper tank can be held in the anaerobic state is obtained. It is preferable that the cell accommodating container (cell culture insert) 2 is attachable and detachable. By removing a second member 13, the space above the gas permeable membrane is allowed to be accessed.

The cell culture device 10 according to the present embodiment includes a first member (insert receiver) 17 for holding the cell accommodating container (insert) 2. The cell culture device 10 further includes a second member 13 having a structure for regulating the gas concentration in the upper space (including an anaerobic state region 1) of the cell accommodating container (insert) 2. In the present embodiment, the space above the insert 2 of the first member 17 has an inverted truncated cone shape in which guide curves of both the first member 17 and the insert 2 overlap each other in accordance with the form of the insert 2. The second member 13 determines an outer shape of the upper side of the device 10. It is preferable that the second member 13 includes a space in which an oxygen scavenger can be installed. The space is connected to the first member 17 and is further connected to the anaerobic state region 1, so that the oxygen concentration in this portion can be decreased.

In a case where cells are cultured using the cell culture device 10 according to the present embodiment, a cell sheet 4 can be formed on the upper surface of the gas permeable membrane (porous membrane) 7. In this case, the space above the gas permeable membrane 7 is defined by the cell sheet 4, the cell accommodating container (culture insert) 2, the first member 17, and the second member 13, and is in a sealed state in which gas does not pass through.

In the cell culture device 10 according to the present embodiment, each device including the insert 2 is exchangeable by removing the second member 13, or the first member 17 and the second member 13. In the cell culture device 10 according to the present embodiment, the second member 13 may have a structure for measuring the gas concentration (a structure capable of mounting a measuring instrument for measuring the gas concentration, or the like). As an example, an oxygen concentration measurement port 12 can be provided.

The second member 13 has a structure for regulating the gas concentration in the upper space of the cell accommodating container. Examples of the structure for regulating the gas concentration in the upper space of the cell accommodating container include a structure for holding an oxygen scavenger 15 and/or a structure for supplying gas having an adjusted concentration.

The oxygen scavenger 15 may be incorporated into the second member 13, or may be incorporated between the first member 17 and the second member 13. In this case, an oxygen concentration indicator 14 may be installed on the oxygen scavenger 15. The structure for supplying gas having an adjusted concentration is not particularly limited.

As an example, Fig. 3 shows a state in which intestinal bacteria 3 and intestinal epithelial cells 4 are co-cultured using the cell culture device 10 according to the present embodiment. With the cell culture device 10 according to the present embodiment, it is possible to reproduce, in vitro, co-culture of the anaerobic and difficult-to-culture intestinal bacteria 3 and the intestinal epithelial cells 4 under inexpensive conditions. In addition, with the cell culture device 10 according to the present embodiment, it is also possible to suitably use the cell culture device 10 for evaluating interaction between the intestinal bacteria 3 and the intestinal epithelial cells 4.

In the cell culture device 10 according to the present embodiment, as shown in Fig. 1, a nitrogen blowing port 11 and an oxygen concentration measurement port 12 are provided on the upper part of the device. The cell culture device 10 according to the present embodiment includes, from the upper side, the second member 13, the oxygen concentration indicator 14, the oxygen scavenger 15, an O-ring 16, a first member (insert receiver) 17, an O-ring 18, an O-ring presser 19, an insert 2, the plate 6, and a spacer 24 for adjusting a culture medium amount. As described above, the present device 10 can be divided along a division surface. By the spacer 24 for adjusting a culture medium amount, the amount of the culture medium solution in the plate can be adjusted to an appropriate amount according to the cells, and it is possible to not use the excessive culture medium.

The cell culture device 10 according to the present embodiment can be disassembled and assembled as shown in Fig. 2. In a case where the members are viewed from the upper part, a bolt 31, an air joint 32, an upper lid (oxygen concentration measurement port) 12, a packing (silicone sheet) 34, a second member (air chamber) 13, an oxygen concentration indicator 14, an oxygen scavenger 15, an O-ring 16, a knock pin 35, a first member (insert receiver) 17, an O-ring 18, an O-ring presser and bolt 19, an insert 2, a spacer 24, and a plate (6-well plate) 6 are provided.

In the cell culture device shown in Fig. 3, the intestinal epithelial cells 4 are located at a boundary between anaerobic conditions and aerobic conditions. A bottom surface of the insert 2 is a porous membrane 7, and only with the insert 2, air in the upper tank and the lower tank can be exchanged, but the upper tank is held in the anaerobic state only after the cells 4 are seeded on the insert 2 and the barrier by the cell sheet is formed. In addition, by co-culturing the intestinal epithelial cells 4 and the bacteria 3, a response of the intestinal epithelial cells 4 can be evaluated.

Fig. 4 shows a state during an exchange of a culture medium, a state during a culture, and a state during a cell observation and a chemical liquid injection, respectively. In the culture state of the present embodiment, a space of 1 mL by liquid volume is secured at the center of the annular spacer 24. In addition, as shown in the image on the upper right, the insert can be pulled out, and the cells inside can be observed or the culture medium can be replaced.

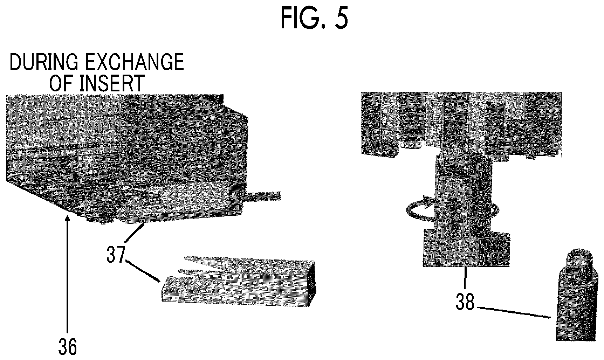

Fig. 5 shows a state during insertion and removal of the insert. The left figure shows a state in which the insert is removed using a drawing tool 37. In addition, the left figure shows a state in which an insert-shaped cover (insert cover 36) for sealing a well portion without the cells is inserted. Furthermore, the right figure shows a state in which the insert is inserted and attached to the first member by an insertion tool 38.

The extent to which the air can be blocked by the barrier by the cell sheet depends on the conditions, but as shown in Examples later, an anaerobic state region 1 and an aerobic state region 5 can be sufficiently separated by seeding the cells 4. In a case where there is a well in which the cells 4 are not seeded, the well can be sealed by attaching the insert cover 36 (Fig. 5).

In the cell culture device according to the present embodiment, an inner space thereof has a two-layer structure of an upper tank and a lower tank with the cell sheet of the gas permeable membrane (intestinal epithelial cell) 4 interposed therebetween. The anaerobic state can be obtained by installing and sealing an oxygen scavenger in the upper tank portion. On the other hand, the lower tank portion has a structure in which the culture medium is put in and the insert is immersed. In this manner, the upper tank seals the lower tank in a manner of being covered with the upper tank, and the lower tank can be maintained in the aerobic state by performing culture in a CO₂ gas incubator or in a multi-gas incubator.

A material of the plate 6 is not particularly limited, and a material generally used in a member for cell culture can be used. For example, a resin material such as polystyrene, polyethylene terephthalate (PET), polypropylene, polycarbonate, and polyamides such as nylon, the resin material having a thickness capable of maintaining gas impermeability, or a metal such as stainless steel can be used.

A material of the cell accommodating container (insert) 2 is not particularly limited, and a material generally used in a member for cell culture can be used. For example, a gas-impermeable resin material such as polystyrene, polyethylene terephthalate (PET), polypropylene, polycarbonate, and polyamides such as nylon can be used.

A material of the first member 17 and the second member 13 is not particularly limited, and for example, a resin material such as polystyrene, polyethylene terephthalate (PET), polypropylene, polycarbonate, and polyamides such as nylon, the resin material having a thickness capable of maintaining gas impermeability, or a metal such as stainless steel can be used.

### <Kit for cell culture>

The cell culture device according to the embodiment of the present invention can be provided as a kit for cell culture in combination with an oxygen scavenger.

The oxygen scavenger is not particularly limited as long as it is a substance having a function of removing oxygen in the air by an oxidation reaction, adsorption, or the like. As the oxygen scavenger, for example, a reducing metal powder such as iron powder, zinc, and tin powder; a lower metal oxide such as iron oxide, ferrous oxide, ferric oxide, cerium oxide, and titanium oxide; a reducing metal compound such as iron carbide, silicon iron, iron carbonyl, and ferrous hydroxide; an inorganic compound such as a hydroxide of alkali metal or alkaline earth metal, a sulfite, and a carbonate; ascorbic acid, erythorbic acid, erucic acid, and salts thereof; gallic acid; a combination of tocopherol and an electron-donating substance; a polymer compound having a polyhydric phenol in the skeleton; a combination of sugars such as glucose, fructose, galactose, maltose, and cellobiose and an electron-donating substance, particularly a combination with a basic substance or sugar oxidases such as glucose oxidase; or the like can be used.

### <Cell culture method>

According to the present invention, there is provided a cell culture method including, using the cell culture device according to the embodiment of the present invention, culturing cells on the gas permeable membrane in a state in which the cells form a cell sheet.

The cells which form the cell sheet are not particularly limited, but are preferably mammalian cells such as human cells. Examples of the cells include epithelial cells (intestinal epithelial cells, oral epithelial cells, vaginal epithelial cells, and the like), Caco-2 cells, HT29 cells, T84 cells, primary intestinal epithelial cells, follicular cells, M cells (microfold cells), goblet cells (mucus-producing cells), endocrine cells, mucous membrane secretory cells, crypt cells, Paneth cells, and intestinal epithelial stem cells. Furthermore, the cells may be epithelial cells that are differentiated and induced from a stem cell, such as an induced pluripotent stem cell (iPS cell) or an embryonic stem cell (ES cell). As the cell, an epithelial cell is preferable, and an intestinal epithelial cell is more preferable. As an example, F-hiSIEC (FUJIFII,M Corporation) which is an intestinal epithelial cell induced to differentiate from the iPS cell can be used.

In the cell culture method according to the embodiment of the present invention, it is preferable to co-culture bacteria on the cell sheet.

The type of the bacteria is not particularly limited, but is preferably bacteria capable of inhabiting a human intestine, and more preferably anaerobic bacteria.

Examples of the bacteria include lactic acid bacillus, lactic acid cocci, and other bacteria.

Examples of the lactic acid bacillus include genus Alkalibacterium, genus Atopobactor, genus Camobacterium, genus Fructobacillus, genus Halolactibacillus, genus Isobaculum, genus Lactobacillus, genus Marinilactibacillus, genus Olsenella, genus Paralactobacillus, genus Pilibacter, and genus Weissella.

Examples of the lactic acid cocci include genus Abiotrophia, genus Bavariicoccus, genus Enterococcus, genus Granulicatella, genus Melissococcus, genus Lacticigenium, genus Lactococcus, genus Pediococcus, genus Tetragenococcus, genus Trichococcus, and genus Vagococcus.

Examples of the other bacteria include genus Bacteroides, genus Ruminococcus, genus Peptococcus., genus Peptostreptococcus, genus Bifidobacterium, genus Escherichia coli, genus Achromobacter, genus Bacillus, genus Enterococcus, genus Clostridium, genus Faecalibacterium, genus Mycoplasma, and genus Salmonella.

In the cell culture method according to the embodiment of the present invention, it is preferable that the cells constituting the cell sheet are cultured under the aerobic conditions, and the bacteria co-cultured on the cell sheet are cultured under the anaerobic conditions.

By co-culturing the intestinal epithelial cells and the bacteria, a response of the intestinal epithelial cells or the bacteria can be evaluated.

The type of a culture medium used in the cell culture is not particularly limited, and depending on the type of the cells, a basal medium such as minimum essential media (MEM), a basal medium eagle (BME) medium, a Dulbecco's modified eagle medium (DMEM), an α-MEM medium, Iscove's modified Dulbecco's media (IMDM), a DM-160 medium, a Fisher medium, an F1 medium, a WE medium, and an RPMI1640 medium can be used. Furthermore, serum (fetal calf serum or the like), various growth factors, antibiotics, amino acids, and the like may be added to the basal medium. A serum-free medium can also be used. A commercially available culture medium such as F-hiSIEC Culture Medium (FUJIFILM Corporation) may be used.

A culture temperature is generally 30°C to 40°C, preferably 32°C to 37°C and more preferably 36°C to 37°C, and the culture temperature may be changed during the culture.

The culture can be performed in an atmosphere having a CO₂ concentration of generally 0% to 40%, preferably 2% to 25%, and more preferably 3% to 20%.

In the present invention, the cell culture device according to the embodiment of the present invention can be installed in a CO₂ gas incubator or in a multi-gas incubator to culture the cells. One of advantages of the present invention is that the co-culture of the cell sheet and the bacteria can be performed without requiring a special device such as an anaerobic chamber.

The present invention will be described in more detail with reference to the following examples, but the present invention is not limited to the examples.

### Examples

### [Example 1]

### <Method>

One gas absorbent (oxygen scavenger) (Anaeropouch or Anaeropack: Mitsubishi Chemical Corporation) was installed in the upper tank of the cell culture device in Fig. 1, the insert cover in Fig. 5 was inserted, and the sealed upper tank portion was installed on the 6-well plate 6, which was the lower tank portion. The entire device was placed in a CO₂ incubator, and an O₂ concentration in the upper tank portion was measured over time by PreSens Measurement Studio 2. The O₂ concentration of the upper tank portion was measured in the same manner as described above even in a case where the gas absorbent (oxygen scavenger) was not installed.

### <Result>

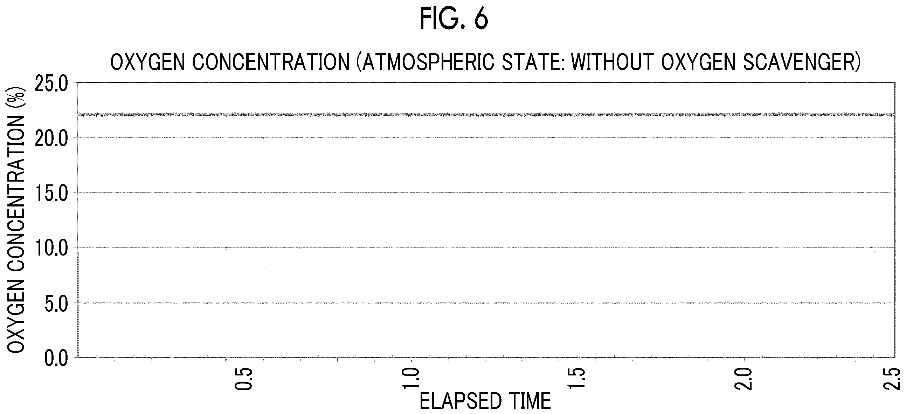

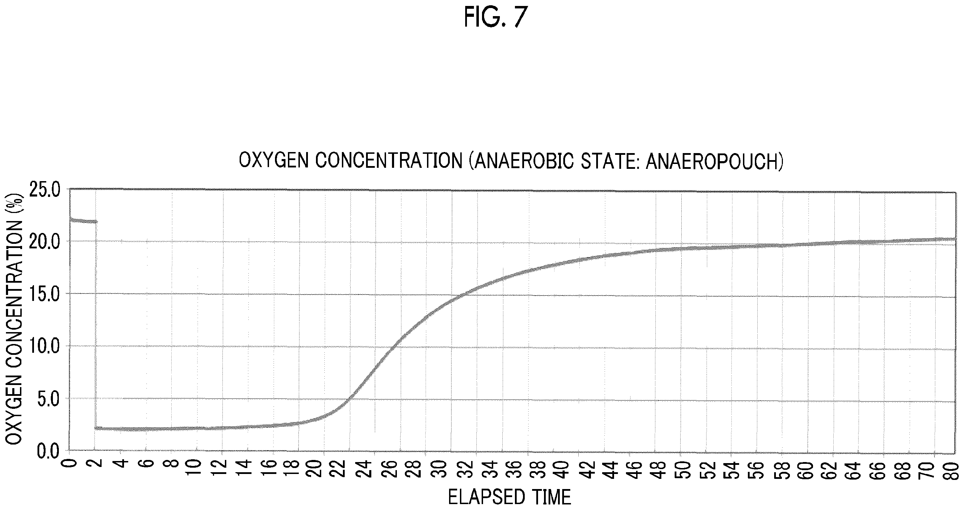

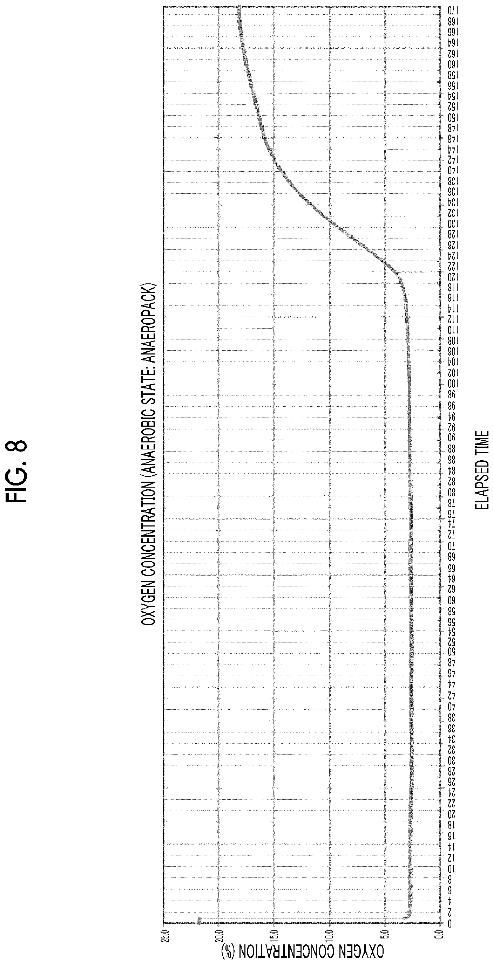

The measurement results are shown in Figs. 6 to 8. In a case where the gas absorbent (oxygen scavenger) was not present, the O₂ concentration was approximately 20% in the same manner as the atmospheric pressure (Fig. 6). On the other hand, in a case where the Anaeropouch was placed, the O₂ concentration of approximately 0% to 3% was maintained for approximately 10 hours (Fig. 7), and in a case where the Anaeropack was placed, the O₂ concentration of approximately 0% to 3% was maintained for approximately 120 hours (Fig. 8).

### [Example 2]

### <Method>

One gas absorbent (oxygen scavenger) (Anaeropack: Mitsubishi Chemical Corporation) was installed in the upper tank of the cell culture device in Fig. 1, the upper tank portion was sealed, and the sealed upper tank portion was installed on the 6-well plate, which was the lower tank portion. In addition, intestinal epithelial cells F-hiSIEC (FUJIFILM Corporation) were seeded on a cell culture insert (Millipore) coated with Matrigel (trade name), and cultured for 9 days according to a manual using F-hiSIEC Culture Medium (FUJIFILM Corporation). 150 µL of F-hiSIEC Culture Medium (FUJIFILM Corporation) was added to an upper surface of the cell culture insert on which the cells on the 9th day of the culture had been seeded, and the cell culture insert was installed in the device from a lower side of the upper tank of the device, using the insertion tool shown in Fig. 5. While sandwiching the cell culture insert, 1 mL of a culture medium was added to a well plate of the lower tank, the upper tank portion of the device was installed in the well plate, and the entire device was placed in a CO₂ incubator. The O₂ concentration in the culture medium on the upper surface of the cells and in the lower tank portion after the incubation was measured by PreSens Measurement Studio 2.

### <Result>

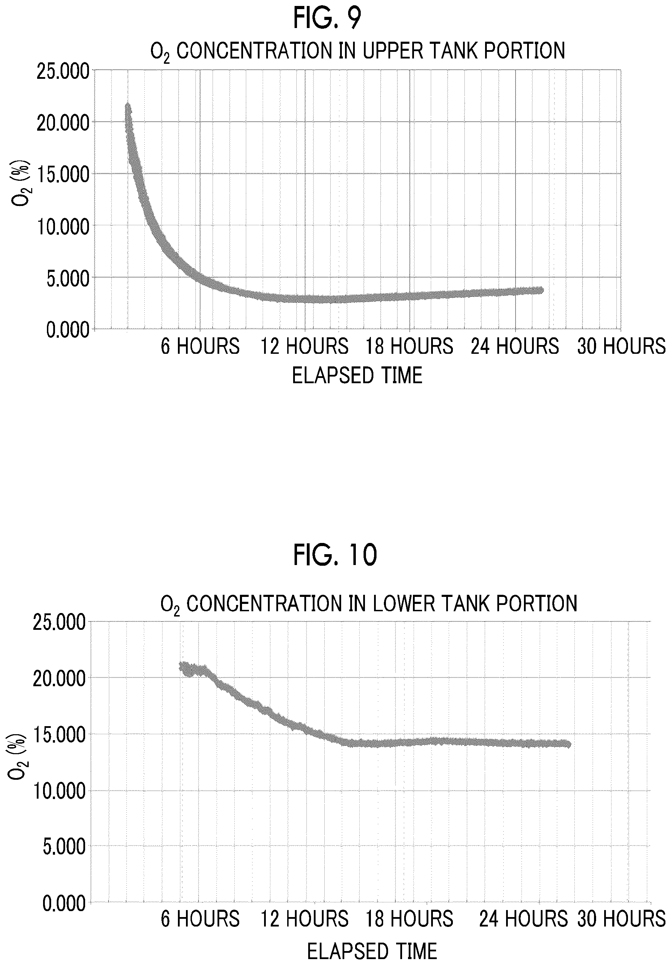

The measurement results are shown in Figs. 9 and 10. The oxygen concentration of the upper tank and the lower tank was respectively 2.9% and 15.2% 12 hours after the start of the incubation, respectively 3.5% and 15.0% 18 hours after the start of the incubation, and respectively 3.7% and 14.2% 24 hours after the start of the incubation.

### [Example 3]

### <Method>

One gas absorbent (oxygen scavenger) (Anaeropack: Mitsubishi Chemical Corporation) was installed in the upper tank of the cell culture device in Fig. 1, the upper tank portion was sealed, and the sealed upper tank portion was installed on the 6-well plate, which was the lower tank portion. In addition, intestinal epithelial cells F-hiSIEC (FUJIFILM Corporation) were seeded on a cell culture insert (Millipore) coated with Matrigel, and cultured for 9 days according to a manual using F-hiSIEC Culture Medium (FUJIFILM Corporation). 150 µL of F-hiSIEC Culture Medium (FUJIFILM Corporation) was added to an upper surface of the cell culture insert on which the cells on the 9th day of the culture had been seeded, and the cell culture insert was installed in the device from a lower side of the upper tank of the device, using the insertion tool shown in Fig. 5. While sandwiching the cell culture insert, 1 mL of a culture medium was added to a well plate of the lower tank, the upper tank portion of the device was installed in the well plate, and the entire device was placed in a CO₂ incubator for culture for 72 hours. After the culture, a trans-epithelial electrical resistance (TEER) value of the intestinal epithelial cells was measured with an EVOM2 system. In addition, the permeation rate (Papp) of Lucifer yellow and the gene expression of various intestinal epithelial cell markers were compared with cells cultured under aerobic conditions in both the upper tank and the lower tank. The aerobic conditions in the upper tank and the lower tank mean cells cultured by installing a lid attached to the well plate without using the co-culture device. In the permeation test of Lucifer yellow, the upper and lower culture media in the insert containing the cells are substituted with HBSS and incubated at 37°C for 1 hour. Thereafter, the solution in the upper part of the cell culture insert was replaced with a 110 µM Lucifer yellow/HBSS solution, and incubated at 37°C. After 1 hour, the amount of substance which had passed through the lower part of the cell culture insert was quantified by a plate reader (Enspire, PerkinElmer Inc.), and the permeation rate and the permeability coefficient (Papp) were calculated.

First, total RNA was extracted from the cells for measuring gene expression. For the RNA extraction, the cells were dissolved in Qiazol (QIAGEN) and 20% chloroform was added thereto, and the supernatant of the solution, obtained by centrifugation at 12,000 g for 10 minutes, was collected. Total RNA was extracted from the supernatant using RNeasy mini kit (QIAGEN) according to the manual. cDNA was synthesized from the total RNA according to the manual using High Capacity RNA-to-cDNA kit (Applied Biosystems). The obtained cDNA was used to measure a gene expression level using Taqman Gene Expression Assay system and Taqman Gene Expression Master Mix (Applied Biosystems) with ViiA7 real-time PCR system (Applied Biosystems). In the comparison of the gene expression level, an expression level of an internal standard gene 18S was corrected by a ΔΔCT method, and the expression in Adult intestine (AI) was set to 100 to indicate a relative value.

### <Result>

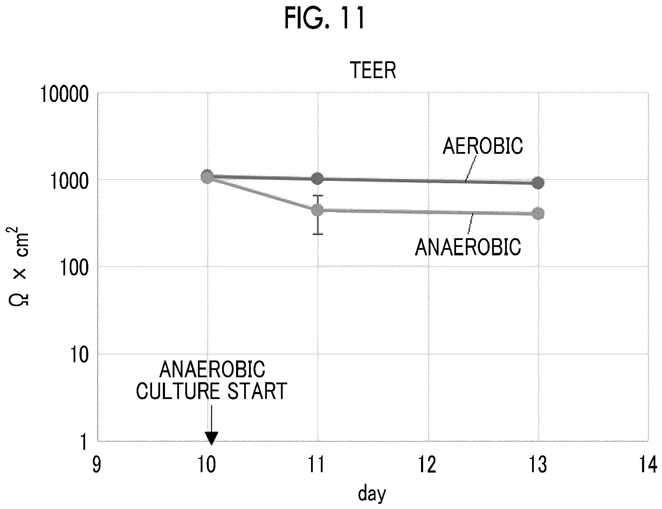

The oxygen concentration 72 hours after the start of the cell culture by the cell culture device according to the embodiment of the present invention was 2.5% in the upper tank and 15% in the lower tank. The TEER value of anaerobic culture cells 72 hours after the start of the culture was slightly lower than that of aerobic culture cells, but was a sufficient resistance value as compared with the biological small intestine (approximately 200 to 300 Ω × cm²) (Fig. 11). In addition, the Papp of Lucifer yellow in the anaerobic cultured cells increased by approximately 2 times as compared with that in the aerobic cells, but the increase did not significantly affect the performance of the cells (Table 1). In addition, there was no significant difference in the gene expression of the intestinal epithelial cell markers between the anaerobic conditions and the aerobic conditions (Table 2).

**[Table 1]**

| Permeability coefficient (Papp) (× 10⁻⁶ cm/sec) | |
|---|---|
| | Average (mean) |
| Aerobic | 0.70 |
| Anaerobic | 1.54 |

**[Table 2]**

| Sample name | Villin | PEPT1/SLC15A | LGR5 | CDX2 | ISX |
|---|---|---|---|---|---|
| Caco-2 | 410 | 36 | 130 | 337 | 213 |
| Adult intestine | 100 | 100 | 100 | 100 | 100 |
| Adult liver | 7 | 12 | 16 | N.D | N.D |
| Aerobic | 109 | 57 | 54 | 187 | 509 |
| Anaerobic | 116 | 42 | 25 | 116 | 313 |

| | | | | | |
|---|---|---|---|---|---|
| N.D: Not Detected | | | | | |

### [Example 4] (Co-culture)

### <Method>

One gas absorbent (oxygen scavenger) (Anaeropack: Mitsubishi Chemical Corporation) was installed in the upper tank of the cell culture device in Fig. 1, the upper tank portion was sealed, and the sealed upper tank portion was installed on the 6-well plate, which was the lower tank portion. In addition, intestinal epithelial cells F-hiSIEC (FUJIFILM Corporation) were seeded on a cell culture insert (Millipore) coated with Matrigel (trade name), and cultured for 9 days according to a manual using F-hiSIEC Culture Medium (FUJIFILM Corporation). On the 7th day of the culture, the culture medium was exchanged with an F-hiSIEC Culture Medium (FUJIFILM Corporation) without antibiotics. The cell culture insert on which the cells on the 9th day of the culture had been seeded was installed in the cell culture device from the lower side of the upper tank of the cell culture device using the insertion tool of Fig. 5. 1 mL of a culture medium was added to a well plate of the lower tank while sandwiching the cell culture insert. On the other hand, intestinal bacteria Bacteroides fragilis (ATCC 25285) were suspended in a liquid culture medium of modified GAM Broth (Nissui), put into a square jar (MITSUBISHI GAS CHEMICAL COMPANY, INC., A-110) containing one Anaeropack, and pre-cultured in a CO₂ incubator at 37°C. In addition, intestinal bacteria Lactococcus Lactis (Riken BioResource Research Center, JM5805) were suspended in a liquid culture medium of a modified GAM Broth (Nissui) and pre-cultured in a CO₂ incubator at 30°C. On the day of co-culture, the number of viable cells was measured using LIVE/DEAD BacLight Bacterial Viability and counting kit (Thermo Fisher Scientific Inc.), and 2 × 10⁵ viable intestinal bacteria were suspended in 300 µL of F-hiSIEC Culture Medium (FUJIFILM Corporation) without antibiotics, and the culture medium in the cell culture insert installed in the cell culture device was replaced. The second member 17 of the cell culture device was set to seal the upper tank portion, and the entire cell culture device was placed in a CO₂ incubator and cultured at 37°C for 48 hours. That is, the upper tank of the cell culture device was in an anaerobic state and the lower tank thereof was in an aerobic state, and the intestinal bacteria and the intestinal epithelial cells were co-cultured. In addition, as a control, the co-culture was performed without using the cell culture device, and the co-culture was performed for 48 hours under the aerobic conditions in both the upper and lower tanks and under the anaerobic conditions in both the upper and lower tanks. Specifically, a cell culture insert was installed in a cell culture plate in which intestinal epithelial cells (F-hiSIEC, FUJIFII,M Corporation) were cultured, F-hiSIEC Culture Medium (FUJIFILM Corporation) that no antibiotics were added and intestinal bacteria were suspended was added to the cell culture insert, and the cell culture insert was cultured in a CO₂ incubator, thereby performing culture under the aerobic conditions in both the upper and lower tanks. In addition, a cell culture insert was installed in a cell culture plate in which intestinal epithelial cells (F-hiSIEC, FUJIFII,M Corporation) were cultured, F-hiSIEC Culture Medium (FUJIFILM Corporation) that no antibiotics were added and intestinal bacteria were suspended was added to the cell culture insert, and the cell culture insert was cultured in a square jar (MITSUBISHI GAS CHEMICAL COMPANY, INC., A-110) containing one Anaeropack, thereby performing culture under the anaerobic conditions in both the upper and lower tanks. After the co-culture, the bacterial suspension was collected, and the number of viable cells and the survival rate were measured again using LIVE/DEAD BacLight Bacterial Viability and counting kit (Thermo Fisher Scientific Inc.).

### <Result>

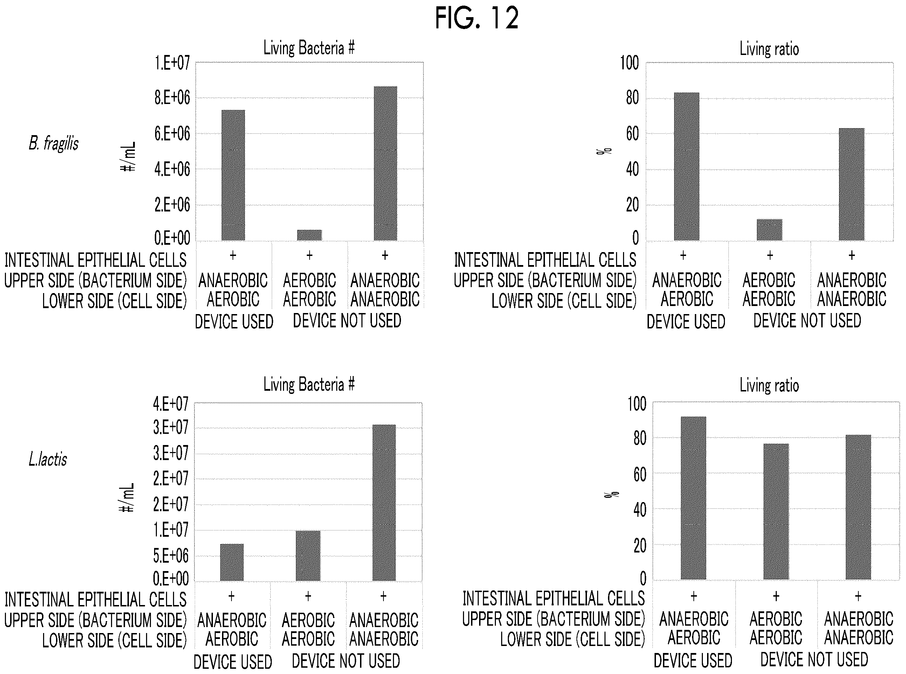

The measurement results are shown in Fig. 12.

As a result of co-culturing Bacteroides fragilis, which is a facultative anaerobic bacterium, with the intestinal epithelial cells using the cell culture device, the number of bacteria after 48 hours of the culture was 7 × 10⁶, and a significant increase in number of bacteria was observed as compared with the number of bacteria added, 2 × 10⁵, and a high survival rate of 83% was obtained. On the other hand, in a case where the culture was performed under the aerobic conditions in both the upper and lower tanks without using the cell culture device, the increase in number of bacteria was not observed, and the survival rate was remarkably low. In addition, in a case where the culture was performed under the anaerobic conditions in both the upper and lower tanks without using the cell culture device, the increase in bacteria was observed, but the survival rate was 63%, which was lower than that in the case where the cell culture device was used.

As a result of co-culturing Lactococcus lactis, which is a transitive anaerobic bacterium, with the intestinal epithelial cells using the cell culture device, the number of bacteria after 48 hours of the culture was 7 × 10⁶, and it was confirmed that the number of bacteria was significantly increased, and the survival rate of the intestinal bacteria was higher than that in the case where the cell culture device was not used.

With the cell culture device according to the embodiment of the present invention, it is possible to reproduce, in vitro, a growth and proliferation environment of anaerobic intestinal bacteria having a difficult-to-culture property in a human body. In addition, it can be used for evaluating the interaction between the intestinal bacteria and the living body, which is industrially useful.

### Explanation of References

1: anaerobic state region
2: cell accommodating container (insert)
3: intestinal bacteria
4: intestinal epithelial cell
5: aerobic state region
6: plate
7: gas permeable membrane
10: cell culture device
11: nitrogen blowing port
12: oxygen concentration measurement port
13: second member
14: oxygen concentration indicator
15: oxygen scavenger
16: O-ring
17: first member
18: O-ring
19: O-ring presser
23: culture medium
24: spacer
31: bolt
32: air joint
34: packing
35: knock pin
36: insert cover
37: drawing tool
38: insertion tool

## Claims

1. A cell culture device comprising:
a plate having a concave portion for accommodating a culture medium;
a cell accommodating container having a gas permeable membrane;
a first member for holding the cell accommodating container; and
a second member having a structure for regulating a gas concentration in an upper space of the cell accommodating container,
wherein, in a case where a cell sheet is formed on an upper surface of the gas permeable membrane, a space above the gas permeable membrane is in a sealed state in which gas does not pass through.

2. The cell culture device according to claim 1,
wherein the cell accommodating container is attachable and detachable.

3. The cell culture device according to claim 1 or 2,
wherein the space above the gas permeable membrane is allowed to be accessed by removing the second member.

4. The cell culture device according to any one of claims 1 to 3,
wherein the structure for regulating the gas concentration in the upper space of the cell accommodating container is a structure for holding an oxygen scavenger and/or a structure for supplying gas having an adjusted concentration.

5. The cell culture device according to any one of claims 1 to 4,
wherein the second member has a structure for measuring the gas concentration in the upper space.

6. A kit for cell culture, comprising:
the cell culture device according to any one of claims 1 to 5; and
an oxygen scavenger.

7. A cell culture method comprising:
culturing cells on the gas permeable membrane in a state in which the cells form a cell sheet, using the cell culture device according to any one of claims 1 to 5.

8. The cell culture method according to claim 7,
wherein the cells are intestinal epithelial cells.

9. The cell culture method according to claim 7 or 8,
wherein bacteria are co-cultured on the cell sheet.

10. The cell culture method according to claim 9,
wherein the bacteria are bacteria capable of inhabiting a human intestine.

11. The cell culture method according to claim 9 or 10,
wherein the cells are cultured in aerobic conditions, and the bacteria are cultured in anaerobic conditions.

12. The cell culture method according to any one of claims 9 to 11,
wherein a response of intestinal epithelial cells or bacteria is evaluated by co-culturing the intestinal epithelial cells and the bacteria.

13. The cell culture method according to any one of claims 7 to 12,
wherein the cells are cultured by installing the cell culture device according to any one of claims 1 to 5 in a CO₂ gas incubator or in a multi-gas incubator.
